# EUROPEAN PATENT APPLICATION

(11) **EP 1 364 672 A1**
(43) Date of publication of application: **26.11.2003**
(21) Application number: 03009879.2
(22) Date of filing: 15.05.2003
(51) Int. Cl.: A61M 16/00, A61B 5/08

(54) **Apparatus for controlling the dispensing of medical gases, particularly for assisting respiration**

(30) Priority: 20.05.2002 IT BO20020310
(71) Applicant: SIM Italia s.r.l., 40018 San Pietro in Casale (Prov. of Bologna) (IT)
(72) Inventor: Righetti, Roberto, 40018 San Pietro in Casale (Pr. Bologna) (IT)
(74) Representative: Modiano, Guido, Dr.-Ing.

(57) **Abstract**

An apparatus for controlling dispensing of medical gases, comprising a unit (2) for supplying medical gas, connected hermetically by way of a tube (3) to a gas dispenser (4) connected to a patient, a detector (7) located along the tube (3) for detecting correct respiration of the patient with at least one pressure sensor (5) that detects cyclic pressure increases inside the dispenser caused by patient expirations, binary output signals being conveyed by the sensor to the detector (7) which compares periodicity of the signals with a reference physiological periodicity, and an alarm indicator (8) controlled by the detector and activated if the periodicity does not occur.

## Description

The present invention relates to an apparatus for controlling the dispensing of medical gases, particularly for assisting respiration.

The administration of medical gases, specifically oxygen, is customary for the treatment of certain disorders, mainly affecting the respiratory system.

The patient breathes through a dispenser that is connected to a centralized or local oxygen supply unit; the dispenser is normally a nasal cannula, through which the patient can receive the oxygen according to two different criteria: dispensing with continuous supply and dispensing on demand. In the first solution, the dispenser supplies oxygen continuously, regardless of the respiratory act that the patient is performing at that moment. In the second solution, a valve is inserted in the gas flow ducts, and when overpressure occurs in the cannula due to patient expiration, the emission of oxygen is interrupted, resuming when the pressure decreases again as a consequence of inspiration.

The risk, for acute patients who must be constantly connected to the dispenser, is that as a consequence of involuntary movements the cannula can shift or escape from the nose, therefore interrupting the administration of oxygen to the patient. The same problem occurs if the gas supply duct through which the oxygen flows to the dispenser undergoes mechanical deformations, preventing the free flow of the gas toward the patient.

The aim of the present invention is to obviate the cited shortcomings and meet the mentioned requirements, by providing an apparatus for controlling the dispensing of medical gases, particularly for assisting respiration, that obviates the interruption of the service provided by the dispenser if such dispenser exits from the nostrils or if the gas feed ducts are blocked.

An object of the present invention is to provide a structure that is simple, relatively easy to provide in practice, safe in use, effective in operation, and relatively low in cost.

This aim and this object, which will become better apparent hereinafter are achieved by the present apparatus for controlling the dispensing of medical gases, particularly for assisting respiration, comprising a centralized or local unit for supplying medical gas, which is connected hermetically by way of a tube to a dispenser of said gas to which the patient is connected, characterized in that along said tube there is a detector for detecting the correct respiration of the patient which has at least one pressure sensor adapted to detect the cyclic increases in pressure inside said dispenser caused by patient expirations, in that said sensor conveys its output signals, of the binary type, to said detector, which is adapted to compare the periodicity of said signals with a reference physiological periodicity, an alarm indicator being controlled by said detector and being activated if said periodicity has not occurred due to obstruction of the tube or extraction of the dispenser.

Further features and advantages of the invention will become better apparent from the detailed description of a preferred but not exclusive embodiment of an apparatus for controlling the dispensing of medical gases, particularly for assisting respiration, according to the invention, illustrated by way of non-limitative example in the accompanying drawings, wherein:

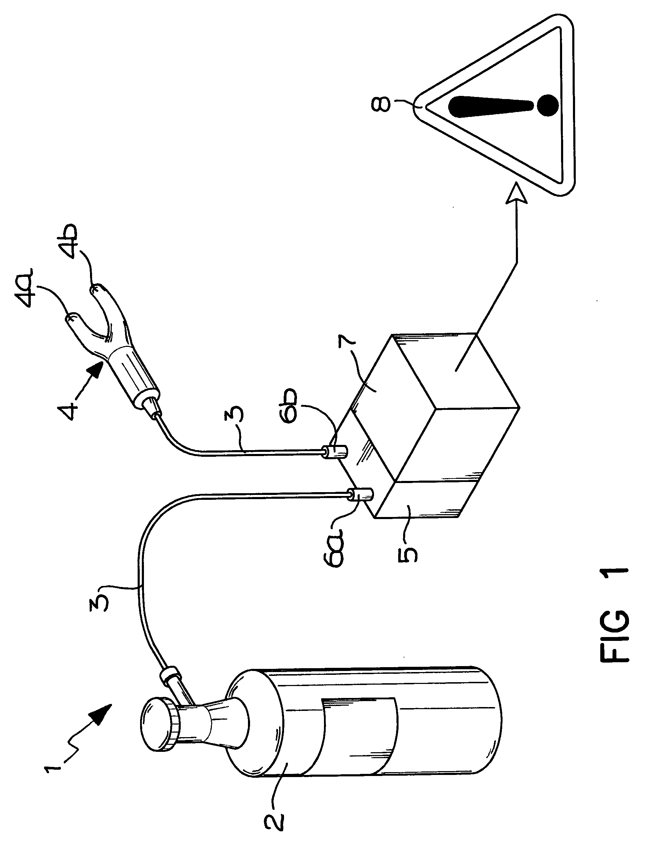

Figure 1 is a view of a control apparatus according to the invention applied to a dispenser of the nasal cannula type;

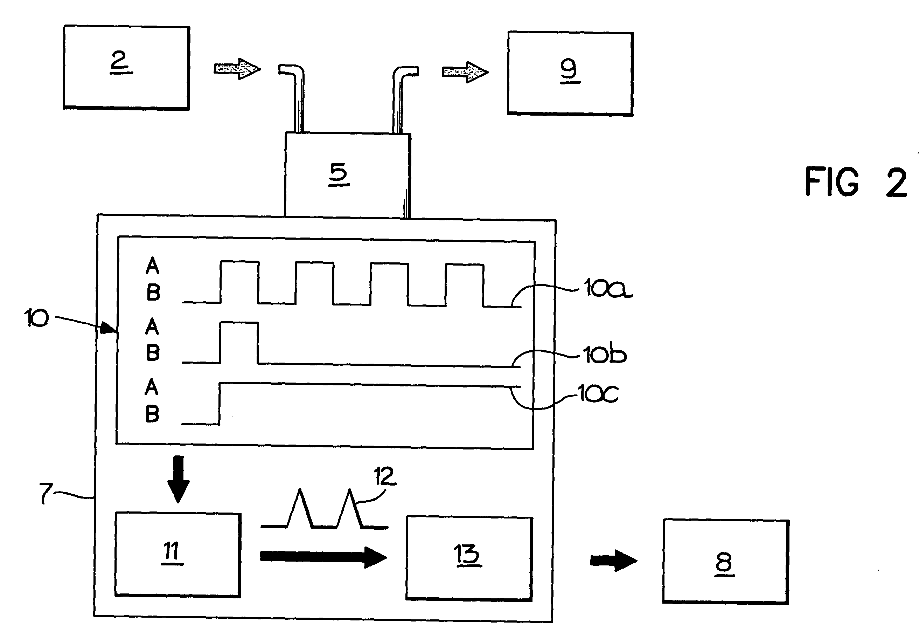

Figure 2 is a functional block diagram of the components of the control apparatus according to the invention.

With reference to the figures, the reference numeral 1 generally designates a device for assisted respiration that is provided with the apparatus for controlling the dispensing of medical gases, particularly for assisting respiration, according to the invention.

The reference numeral 2 designates a local unit for supplying medical gas, in the illustrated case a bottle that contains said gas, on the top of which an end of a tube 3 is connected at the outflow control valves. The dispenser, which in the particular case is a nasal cannula 4, is fixed to the opposite end of the tube 3, maintaining the continuity of the internal free cross-section for the passage of the gas; the cannula 4, during use, is arranged so that its two tubular tips 4a and 4b, arranged at the opposite end with respect to the end for fixing to the tube 3, are inserted in the nostrils of the patient 9. In its central section, the tube 3 is provided with the pressure sensor 5: the tube 3 leads to the sensor by means of an inlet duct 6a and exits from it, after being sampled, through the outlet duct 6b. The sensor 5 is connected electrically to the detector 7, for detecting correct respiration of a patient, which drives the alarm indicator 8.

The detector 7 is constituted by a signal conversion unit 11 and by a timer 13.

The signal conversion unit 11 is suitable to detect the rising and falling fronts of the signal 10 provided by the sensor 5 and to emit, at each one of said fronts, a peak pulse as an output signal 12.

The timer 13 behaves like a conventional timer in which, once the time to be counted has been set and once it has been activated, the input signal is the signal emitted by the unit 11, i.e., the signal 12. The set time depends on a certain reference physiological periodicity, which is typical of respiratory acts and can be deduced from medical treatises. The count is reset at each pulse received from the unit 11.

When the timer 13 is able to perform the entire count, it emits an output signal, which reaches the alarm indicator 8.

The alarm indicator 8 can be of the acoustic type, such as a buzzer or bell, or of the visual type, such as a flashing light or luminous sign, or can be controlled by a computer suitable for monitoring, on the screen of which the message indicating the anomaly is displayed.

The indicator 8 can be arranged either in the same room in which the patient is located, reporting the occurred anomaly to said patient, or in a manned room, which is distant with respect to the room in which the patient is located, such as the paramedic reception room in the case of hospitals, ensuring timely intervention if a signal occurs.

Operation of the invention is as follows: once the patient 9 has been arranged so that the cannula 4 is inserted in his nostrils, pressure variations occur inside the sensor 5 according to the cycle of respiratory acts.

Both when the medical gas dispensing system is of the continuous-supply type and when it is of the on-demand type, the overpressures and negative pressures caused by respirations can be detected if the sensor 5 is set quantitatively for the intended specific purpose.

Upon inspiration, the sensor 5 detects a reduction in the pressure inside the tube 3 and therefore outputs the value that corresponds to the low level B of the binary logic in use.

When the patient 9 breathes out, the sensor 5 detects an increase in the pressure inside the tube 3 and emits a signal that corresponds to the high level A of the binary logic in use.

The succession of respiratory acts therefore entails that the output signal 10 of the sensor 5 is, in regular operating conditions, a square wave 10a.

The succession of rising or falling fronts of the level of the signal 10 is converted by the unit 11 into a series of peak pulses 12, each pulse occurring at each front.

The time between each peak and the next, in the case of regular operation, is such that the timer 13 is reset at every count before it can reach the end.

The preset time counted by the timer 13 must in fact be such as to allow to skip a few respiratory acts, for example because the patient 9 is talking and is breathing through his mouth, without activating the alarm indicator 8.

The low level B also corresponds to the case in which the patient 9 intentionally or unintentionally removes the cannula 4 or if expiration does not occur after inspiration, as indicated by the signal 10b.

In this case, the absence of state change fronts in the signal 10 also entails that the outlet 12 of the unit 11, starting from the moment when the problem occurs, is also zero, and therefore there is no succession of pulses. Therefore, since the timer 13 is not reset by said pulses, it can continue the count, activating the alarm indicator 8 when the count ends.

Likewise, in the case of an obstructed cannula 4 a certain overpressure is kept constant inside the tube 3. Said overpressure is detected by the sensor 5, which keeps its output 10c at the high level A indefinitely, until the obstruction is eliminated.

Persistence of the signal 10 at the high value A entails the absence of state change fronts and therefore the absence of pulses downstream of the unit 11 in the signal 12. In this case also, the timer 13 can complete the count and activate the alarm indicator 8.

If an oxygen-dependent patient unintentionally removes the dispensing nasal cannula, he is warned locally by the alarm indicator; the advantage of this use is the fact that the cannula generally slides out unintentionally during sleep. If the control apparatus is not present, the patient might wake up and realize that the cannula has slid out only as a consequence of the onset of the symptoms caused by lack of oxygenation; the presence of the control apparatus according to the invention entails that the patient is woken up shortly after the cannula has slid out, thus avoiding the sickness caused by hypoxia.

The invention thus conceived is susceptible of numerous modifications and variations, all of which are within the scope of the appended claims.

It has thus been shown that the invention achieves the proposed aim and objects.

For example, if an on-demand dispensing system is used, it is possible to activate a second sensor, which upon inspiration is activated regularly, resetting the timer every time. If no inspiration occurs within the preset time, the alarm is tripped.

All the details may further be replaced with other technically equivalent ones.

In practice, the materials used, as well as the shapes and the dimensions, may be any according to requirements without thereby abandoning the scope of protection of the appended claims.

The disclosures in Italian Patent Application No. BO2002A000310 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An apparatus for controlling the dispensing of medical gases, particularly for assisting respiration, comprising a centralized or local unit (2) for supplying medical gas, which is connected hermetically by way of a tube (3) to a dispenser (4) of said gas to which the patient is connected, **characterized in that** along said tube (3) there is a detector (7) for detecting the correct respiration of the patient which has at least one pressure sensor (5) adapted to detect the cyclic increases in pressure inside said dispenser caused by patient expirations, **in that** said sensor (5) conveys its output signals, of the binary type, to said detector (7), which is adapted to compare the periodicity of said signals with a reference physiological periodicity, an alarm indicator (8) being controlled by said detector and being activated if said periodicity has not occurred due to obstruction of the tube or extraction of the dispenser.

2. The apparatus according to claim 1, **characterized in that** said detector is constituted by a signal conversion unit (11) for conversion from a binary signal to a peaking signal, and by a timer (13), which is adapted to detect the time elapsing between two successive peaks, activating the alarm indicator for times exceeding a certain preset value.

3. The apparatus according to claim 2, **characterized in that** said conversion unit (11) converts every change of state of the binary signal, emitted by said at least one sensor (5) and received in input, into a peak in its output signal.

4. The apparatus according to claim 2, **characterized in that** said timer (13) receives in input the peaking signal emitted by said conversion unit (11) and **in that** at each peak the time measured starting from the preceding peak is reset.

5. The apparatus according to claim 1, **characterized in that** said medical gas is oxygen.

6. The apparatus according to claim 1, **characterized in that** said dispenser (4) is a nasal cannula.

7. The apparatus according to claim 1, **characterized in that** said dispenser can be equally of the type with continuous dispensing or of the type with on-demand dispensing.

8. The apparatus according to claim 1, **characterized in that** in said binary output signals, one signal level is associated with the absence of overpressure inside said dispenser (4) (inspiration), and the other level is associated with the presence of overpressure inside said dispenser (4) (expiration).

9. The apparatus according to claim 1, **characterized in that** said alarm indicator (8) is located directly on the spot or in other manned rooms and is suitable to report the occurrence of an anomaly detected in the succession of respiratory acts.
